# EUROPÄISCHE PATENTSCHRIFT

(11) **EP 2 656 820 B1**
(45) Veröffentlichungstag und Bekanntmachung des Hinweises auf die Patenterteilung: **29.04.2020**
(21) Anmeldenummer: 13166013.6
(22) Anmeldetag: 23.09.2009
(51) Int. Cl.: A61F 5/00

(54) **Vorrichtung zum Stabilisieren von Körpersegmenten**
Device for stabilizing body segments
Dispositif de stabilisation de segments de corps

(30) Priorität: 26.09.2008 DE 202008012828 U
(43) Veröffentlichungstag der Anmeldung: 30.10.2013
(62) Teilanmeldung aus: 09768323.9
(73) Patentinhaber: Gröhninger, Frank Friedrich, 66450 Oberbexbach (DE)
(72) Erfinder: Gröhninger, Frank Friedrich, 66450 Oberbexbach (DE)
(74) Vertreter: Vièl, Christof

(56) Entgegenhaltungen:
- US-A- 5 628 721
- US-A- 5 695 452
- US-A- 5 792 084
- US-A1- 2007 167 895

## Beschreibung

Die Erfindung betrifft eine Vorrichtung zum Stabilisieren von Körpersegmenten, wobei die Vorrichtung ein oder mehrere an das jeweilige Körpersegment angrenzende weitere Körpersegmente und das bzw. die dazwischen liegenden Gelenke mit abdeckt.

Aus der US 6,779,282 B2 ist ein Sportstrumpf mit einer sich von den Zehen bis zur Ferse erstreckenden Einlage bekannt, die eine steigbügelartige Vorrichtung in Form zweier miteinander verbundener Streifen aus geeignetem Material umfaßt, welche miteinander verbunden sind, um den Knöchel zu stabilisieren und die ein senkrecht zu den Streifen angeordnetes horizontal umlaufendes Band aufweist, das zu einer Bewegungsbeschränkung des Talocalcaneonavikulargelenks und Stabilisieren des Knöchels dient. Hierbei ist vorgesehen, daß die Einlage mit dem Sportstrumpf unter anderem durch Sprühbinden mit Kunststoff verbunden ist. Es kann auch eine Kunststoffschicht auf den beiden Streifen vorgesehen sein. Bei Verwendung dieser Vorrichtung kann das "Tapen", d.h. das Stabilisieren mit Tape-Band entfallen.

Aus der US 5,695,452 A ist eine orthopädische Stützvorrichtung bekannt, der aus einem in Teilbereichen druckgeformten Schaummaterial besteht, wobei die Teilbereiche eine andere Dicke und Dichte aufweisen als die übrigen Bereiche.

Die US 2007/0167895 A1 beschreibt eine orthopädische Stützvorrichtung für ein Gelenk, die aus einer Schicht aus flexiblem Material besteht und die um einen Teil des Körpers gelegt werden kann, wobei das distale und das proximale Ende miteinander verbunden werden.

Die US 5,792,084 A beschreibt eine Kniegelenkstütze mit einer Öffnung und einer aufblasbaren Kammer.

Aus der US 5,628,721 A ist eine Rückenstützvorrichtung bekannt, die um den Körper gelegt wird und bei der über ein aufblasbares Kissen Druck auf den unteren Rücken ausgeübt werden kann.

Es sind weiterhin Gipsverbände zum Festlegen von Körpersegmenten bekannt, welche weitgehend im Bereich der Medizin, insbesondere bei Knochenbrüchen, zur Anwendung kommen. Diese müssen jedoch individuell angelegt werden, was aufwendig ist.

Es sind schließlich auch Kunststoffschalverbände bekannt, welche ebenfalls im Bereich der Medizin verwendet werden und in Anbetracht ihres geringeren Gewichtes den Gipsverbänden zunehmend bevorzugt werden.

Aufgabe der Erfindung ist es, insbesondere für die Anwendung im Bereich des Sports und der Rehabilitationsmedizin eine neuartige Vorrichtung zum Stabilisieren von Körpersegmenten zu schaffen, die als vorgefertigte Konfektionsware oder auch als halbfertige Maßkonfektion vertrieben werden kann.

Diese Aufgabe wird erfindungsgemäß dadurch gelöst, daß auf eine textile Trägerschicht, welche im Wesentlichen die Form des zu stabilisierenden Körpersegmentes aufweist, eine die textile Trägerschicht in ihrer Form stabilisierende Kunststoffschicht aufgespritzt ist, wobei die Kunststoffschicht aufgrund ihrer Verbindung mit der textilen Trägerschicht unverrückbar positioniert ist und wobei mit der Vorrichtung Bewegungen der verschiedenen Gelenke ganz oder teilweise einschränkbar, stabilisierbar oder unterstützbar sind.

Es wird im Rahmen der Erfindung ein funktionelles anatomisch kongruent an den jeweiligen Körpersegmenten anliegendes orthopädisches Hilfsmittel geschaffen. Während bei derzeit am Markt erhältlichen Bandagen eine natürliche konkave Körperüberspannung (beispielsweise im Bereich der Lendenlordose) hervorgerufen wird, so daß dort zur Druckausübung eine Pelotte eingesetzt werden muß, folgt die erfindungsgemäße Vorrichtung in allen Bereichen, auch bei konkaven Körperüberspannungen der Körperkontur, wodurch eine bessere Paßform erreicht wird, und ein unerwünschter Druck auf Körperbereiche durch Pelotten oder dergleichen vermieden wird. Vielmehr kann individuell eingestellt werden, wo Druck auf den Körper ausgeübt werden soll und wo nicht. Auf diese Weise wird eine Vorrichtung zum Stabilisieren von Körpersegmenten geschaffen, die als Orthesen-Bandage oder als Bandagen-Orthese bezeichnet werden kann und bei geringem Gewicht einen hohen Tragekomfort aufweist, da die textile Trägerschicht der Haut des Benutzers zugewandt ist. Mit dieser Vorrichtung können Bewegungen der verschiedenen Gelenke ganz oder teilweise eingeschränkt, stabilisiert oder unterstützt werden, was auch zur Erhöhung der natürlichen Biodynamik genutzt werden kann.

Die stabilisierende Kunststoffschicht kann ein- oder mehrlagig ausgebildet sein. Die Kunststoffschicht ist aufgrund ihrer Verbindung mit der textilen Trägerschicht unverrückbar positioniert. Sie kann mit einer Rutschhilfe abgedeckt werden. Es ist selbstverständlich möglich, zusätzlich weiche Kunststoffapplikationen, beispielsweise aus Silikon, als Pelotte (Druckpolster) auf der Vorrichtung anzuordnen, um gezielt Druck auf bestimmte Körperregionen auszuüben.

Es versteht sich, daß nicht nur das angrenzende Gelenk mit von der Vorrichtung abgedeckt wird, sondern mindestens teilweise auch das an dieses Gelenk angrenzende weitere Körpersegment. Bei einer Vorrichtung, die zur Stabilisierung des Oberkörpers und eines Arms vorgesehen ist, wäre somit der Oberkörper zumindest teilweise, das Schultergelenk und ein Teil des Oberarmknochens abgedeckt.

Es liegt im Rahmen der Erfindung, daß die Kunststoffschicht außen oder innen auf die textile Trägerschicht aufgespritzt ist.

Erfindungsgemäß ist vorgesehen, daß die Körpersegmente die oberen oder unteren Gliedmaßen oder der Rumpf sind.

Es liegt im Rahmen der Erfindung, daß die textile Trägerschicht ein textiles Gewirk oder ein Gewebe ist.

Grundsätzlich kommen beide Alternativen in Frage. Die textile Trägerschicht kann auch elastisch ausgebildet sein, beispielsweise als elastisches Textilgewirk oder -gewebe oder als Textilgummigewirk oder Kombination derselben. Grundsätzlich kommen auch textilähnliche Strukturen, wie beispielsweise Neopren in Frage.

Eine Weiterbildung der Erfindung besteht darin, daß die Vorrichtung mehrlagig aufgebaut ist.

Die Vorrichtung kann starr, elastisch oder flexibel ausgebildet sein.

Es ist möglich, die Vorrichtung nicht nur einlagig, sondern auch mehrlagig auszubilden.

In diesem Zusammenhang ist es möglich, daß Mittel zur Beschränkung des Bewegungswinkels des bzw. der Gelenke vorgesehen sind.

Ebenfalls im Rahmen der Erfindung liegt es, daß mindestens ein Stabilisierungsband zum Erhöhen der Haftung vorgesehen ist.

Ein solches Stabilisierungsband kann sowohl statisch fest als auch elastisch fixierend oder diese beiden Eigenschaften kombinierend ausgebildet sein.

Eine Weiterbildung der Erfindung besteht darin, daß ein Einlegestück, insbesondere aus Metall oder aus Kunststoff, von der Kunststoffschicht umgeben ist.

Ein solches Einlegestück kann beispielsweise aus einem biegbaren Metallelement bestehen, das es ermöglicht, die dann noch formbare Vorrichtung nach dem eigentlichen Herstellungsvorgang individuell formgebend einem Körperteil anzupassen. oder einem Metallelement mit einer einem Körperteil angepaßten Formgebung

Ebenfalls ist es erfindungsgemäß vorgesehen, daß an der dem Körper des Benutzer zugewandten Innenseite eine über ein Ventil mit Luft befüllbare Druckkammer vorgesehen ist.

Durch das Aufpumpen einer solchen Druckkammer kann das Anliegen der Vorrichtung am Körper noch verbessert werden, durch Luftablassen können Schwellungen kompensiert werden oder aber es kann Druck auf die stabilisierten Körpersegmente ausgeübt werden.

Nachfolgend wird ein Ausführungsbeispiel der Erfindung anhand von Zeichnungen beschrieben.

Es zeigt
- Fig. 1: eine schematische Darstellung des Aufbaus der Vorrichtung,
- Fig. 2: eine schematische Darstellung einer erfindungsgemäßen Vorrichtung zur Stabilisierung von Ober- und Unterschenkel und das damit verbundene Kniegelenk in M-L, A-P und Rotationsbewegung.

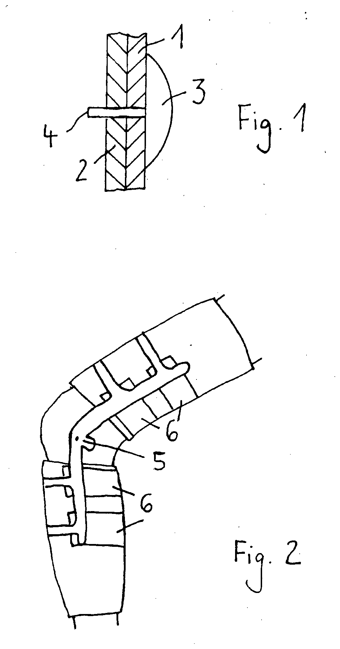

Wie aus Fig. 1 ersichtlich, weist die erfindungsgemäße Vorrichtung eine der Form des bzw. der betroffenen Körpersegmente angepaßte textile Trägerschicht 1 auf, bei der es sich um ein textiles Gewirk oder ein textiles Gewebe handeln kann. Auf diese textile Trägerschicht 1 ist eine die textile Trägerschicht 1 in ihrer Form stabilisierende Kunststoffschicht 2 aufgetragen ist. Hierbei kann es vorgesehen sein, daß in bestimmten hoch beanspruchten Bereichen oder über die gesamte Vorrichtung hinweg dieser Schichtaufbau sandwichartig wiederholt wird, bis die gewünschte mechanische Belastbarkeit erreicht ist.

Weiterhin kann die Vorrichtung eine mit Luft befüllbare Druckkammer 3 aufweisen, die es ermöglicht, den Sitz der Vorrichtung am betreffenden Körpersegment zu verbessern oder aber während des Tragens durch Druckablassen den Sitz der Vorrichtung an dem Körpersegment etwas zu lockern. Diese Druckkammer 3 weist ein Ventil 4 auf, über das die Druckkammer 3 befüllt bzw. entleert werden kann.

Die dargestellte Vorrichtung weist ebenfalls Mittel 5 zur Beschränkung des Bewegungswinkels des Kniegelenks auf, die als mit beiden Teilen der Vorrichtung verbundenes Gelenk 5 zwischen dem den Oberschenkel umfassenden und dem den Unterschenkel umfassenden Teil der Vorrichtung ausgebildet sind, welches einen einstellbaren maximalen Beugewinkel aufweist. Die Fixierbänder 6 können in horizontaler Richtung bzw. in jeder anderen Lagenform angebracht sein.

## Patentansprüche

1. Vorrichtung zum Stabilisieren von Körpersegmenten, wobei die Vorrichtung ein oder mehrere an das jeweilige Körpersegment angrenzende weitere Körpersegmente und das bzw. die dazwischen liegenden Gelenke mit abdeckt, **dadurch gekennzeichnet, daß** auf eine textile Trägerschicht (1), welche die Form des zu stabilisierenden Körpersegmentes aufweist, eine die textile Trägerschicht (1) in ihrer Form stabilisierende Kunststoffschicht (2) aufgespritzt ist, wobei die Kunststoffschicht (2) aufgrund ihrer Verbindung mit der textilen Trägerschicht unverrückbar positioniert ist und wobei mit der Vorrichtung Bewegungen der verschiedenen Gelenke ganz oder teilweise einschränkbar, stabilisierbar oder unterstützbar sind.

2. Vorrichtung gemäß Anspruch 1, **dadurch gekennzeichnet, daß** die Kunststoffschicht außen auf die textile Trägerschicht aufgespritzt ist.

3. Vorrichtung gemäß Anspruch 1, **dadurch gekennzeichnet, daß** die Kunststoffschicht innen auf die textile Trägerschicht aufgespritzt ist.

4. Vorrichtung gemäß Anspruch 1, **dadurch gekennzeichnet, daß** die Körpersegmente die oberen oder unteren Gliedmaßen oder der Rumpf sind.

5. Vorrichtung gemäß Anspruch 1, **dadurch gekennzeichnet, daß** die textile Trägerschicht (1) ein textiles Gewirk oder ein Gewebe ist.

6. Vorrichtung gemäß Anspruch 1, **dadurch gekennzeichnet, daß** die Vorrichtung mehrlagig aufgebaut ist.

7. Vorrichtung gemäß Anspruch 1, **dadurch gekennzeichnet, daß** mindestens ein Stabilisierungsband zum Erhöhen der Haftung vorgesehen ist.

8. Vorrichtung gemäß Anspruch 1, **dadurch gekennzeichnet, daß** Mittel zur Beschränkung des Bewegungswinkels des bzw. der Gelenke vorgesehen sind.

9. Vorrichtung gemäß Anspruch 1, **dadurch gekennzeichnet, daß** ein Einlegestück, insbesondere aus Metall oder aus Kunststoff, von der Kunststoffschicht umgeben ist.

10. Vorrichtung gemäß Anspruch 1, **dadurch gekennzeichnet, daß** an der dem Körper des Benutzers zugewandten Innenseite eine über ein Ventil mit Luft befüllbare Druckkammer vorgesehen ist.

## Claims

1. Device for stabilizing body segments, wherein the device also covers one or more further body segments adjacent to the respective body segment and the joint or joints lying therebetween, **characterized in that** a plastic layer (2) stabilizing the shape of the textile carrier layer (1) is sprayed onto a textile carrier layer (1) which has the shape of the body segment to be stabilized, the plastic layer (2) being immovably positioned due to its connection to the textile carrier layer and wherein movements of the various joints can be fully or partially restricted, stabilized or supported by the device.

2. Device according to claim 1, **characterized in that** the plastic layer is sprayed onto on the outside of the textile carrier layer.

3. Device according to claim 1, **characterized in that** the plastic layer is sprayed on the inside of the textile carrier layer.

4. Device according to claim 1, **characterized in that** the body segments are the upper or lower limbs or the torso.

5. Device according to claim 1, **characterized in that** the textile substrate (1) is a knitted textile fabric or a woven textile fabric.

6. Device according to claim 1, **characterized in that** the device is made up of a plurality of layers.

7. Device according to claim 1, **characterized in that** at least one stabilizing strap is provided to enhance adhesion.

8. Device according to claim 1, **characterised in that** means are provided for limiting the angle of movement of the joint(s).

9. Device according to claim 1, **characterised in that** an insert, in particular made of metal or plastic, is surrounded by the plastic layer.

10. Device according to claim 1, **characterized in that** on the inside of the device, facing the wearer's body, a pressure chamber (3) is provided which can be inflated with air via a valve.

## Revendications

1. Dispositif pour stabiliser des segments corporels, dans lequel le dispositif recouvre un ou plusieurs autres segments corporels adjacents au segment corporel respectif et à l'articulation ou aux articulations situées entre eux, **caractérisé en ce qu'**un couche en matière plastique (2) est pulvérisée sur une couche support textile (1) présentant la forme du segment corporel à stabiliser, la couche en matière plastique (2) stabilisant la forme de la couche support textile, dans laquelle la couche en matière plastique (2) est positionnée de manière fixe en raison de sa liaison avec la couche support textile, et dans laquelle les mouvements des différentes articulations peuvent être entièrement ou partiellement limités, stabilisés ou supportés par le dispositif.

2. Dispositif selon la revendication 1, **caractérisé en ce que** la couche de matière plastique est pulvérisée sur la partie extérieure de la couche support textile.

3. Dispositif selon la revendication 1, **caractérisé en ce que** la couche de matière plastique est pulvérisée sur la partie intérieure de la couche support textile.

4. Dispositif selon la revendication 1, **caractérisé en ce que** les segments du corps sont les membres supérieurs ou inférieurs ou le torse.

5. Dispositif selon la revendication 1, **caractérisé en ce que** la couche support textile (1) est un tricot textile ou un tissu.

6. Dispositif selon la revendication 1, **caractérisé en ce que** le dispositif est constitué de plusieurs couches.

7. Dispositif selon la revendication 1, **caractérisé en ce qu'**au moins une bande de stabilisation est prévue pour augmenter l'adhésion.

8. Dispositif selon la revendication 1, **caractérisé en ce que** des moyens sont prévus pour limiter l'angle de mouvement du ou des articulations.

9. Dispositif selon la revendication 1, **caractérisé en ce qu'**un insert, en particulier en métal ou en matière plastique, est entouré par la couche plastique.

10. Dispositif selon la revendication 1, **caractérisé en ce qu'**un compartiment de pression (3) pouvant être rempli d'air par le biais d'une valve est prévu sur la face interne dirigée vers le corps de l'utilisateur.
